# EUROPEAN PATENT APPLICATION

(11) **EP 1 364 611 A1**
(43) Date of publication of application: **26.11.2003**
(21) Application number: 03011710.5
(22) Date of filing: 23.05.2003
(51) Int. Cl.: A61B 1/005, A61M 25/00

(54) **Flexible endoscope insertion shaft**

(30) Priority: 23.05.2002 US 382836 P; 02.04.2003 US 405187
(71) Applicant: Karl Storz Imaging Inc., Goleta, CA 93117 (US)
(72) Inventor: Guerra, David J., Putnam, CT 06260 (US); Barry, James P., Charlton, Massachusetts (US)
(74) Representative: Heuckeroth, Volker, Dr.

(57) **Abstract**

These and other objects of the present invention are achieved by provision of a flexible endoscope insertion shaft having a hollow inner shaft portion formed from a superelastic metal alloy. The hollow inner shaft portion includes a plurality of longitudinally and radially spaced apart holes passing through a wall thereof.
Flexibility properties of the insertion shaft are variable without varying the thickness of or the composition of the wall of the hollow inner shaft portion by varying a number of, size of, and/or relative position of the plurality of holes. A polymeric coating coats a surface of the hollow inner shaft portion.

## Description

### Related Applications

This patent application claims the benefit of, under Title 35, United States Code, Section 119(e), U.S. Provisional Patent Application No. 60/382,836, filed May 23, 2002.

### Field of the Invention

The present invention relates to a flexible endoscope insertion shaft, and more particularly to a flexible endoscope insertion shaft at least a portion of which is formed from a superelastic alloy material.

### Background of the Invention

Conventional flexible endoscope insertion shafts are fabricated by laying up several different types of materials into a composite structure. These components generally consist of traditional metals, plastics, and adhesives, with the object of achieving a flexible, torsionally stable and crush resistant structure. However, when using conventional components, these objects are not always achieved. For example, conventional shafts may only be guided around large radii of curvature, and may not be capable of elastically returning to their original position when removed from a patient's body. Moreover, known shafts using conventional materials are prone to being crushed, kinked or the like. If the shafts are reinforced to minimize the risk of such occurrences, the shafts are rendered bulky and require a larger than desired outer diameter.

Attempts have been made to Improve upon the design of conventional flexible endoscope insertion shafts. U.S. Patent No. 5,683,348 ("the '348 patent") represents such an attempt. The '348 patent discloses a shank made from a continuous walled, one-piece, tubular superelastic material which is so dimensioned that the endoscope is automatically restored to its original shape by mechanical elasticity when the endoscope is removed from a body. Although this design purports to overcome the deficiencies of conventional shafts, it suffers from a number of disadvantages of its own. One such deficiency is that such a continuous walled structure requires that the dimensions of the tube and the composition of the superelastic material must be carefully controlled in order to achieve the desired flexibility and kink resistance characteristics. Similarly, if it is desired to vary the flexibility of the shaft, for example, for endoscopes to be used in different applications, a new shaft having different dimensions and/or different material compositions must be designed for each such application. Furthermore, since the tube is continuous, it is not possible to provide different portions of the same tube with varying flexibility properties.

What is desired, therefore, is a flexible endoscope insertion shaft which is flexible, torsionally stable and crush resistant, which may be guided around relatively small radii of curvature, which is capable of elastically returning to its original position when removed from a patient's body, which is not prone to being crushed, kinked or the like, which does not require that the dimensions and/or composition of the shaft to be carefully controlled in order to achieve the desired flexibility and kink resistance characteristics, which allows for the flexibility of the shaft to be more easily varied for endoscopes to be used in different applications, and which allows for the flexibility of different portions of the same shaft to be varied.

### Summary of the Invention

Accordingly, it is an object of the present invention to provide a flexible endoscope insertion shaft which is flexible, torsionally stable and crush resistant.

Another object of the present invention is to provide a flexible endoscope insertion shaft having the above characteristics and which may be guided around relatively small radii of curvature.

A further object of the present invention is to provide a flexible endoscope insertion shaft having the above characteristics and which is capable of elastically retuming to its original position when removed from a patient's body.

Still another object of the present invention is to provide a flexible endoscope insertion shaft having the above characteristics and which is not prone to being crushed, kinked or the like.

Yet a further object of the present invention is to provide a flexible endoscope insertion shaft having the above characteristics and which does not require that the dimensions and/or composition of the shaft to be carefully controlled in order to achieve the desired flexibility and kink resistance characteristics.

Still another object of the present invention is to provide a flexible endoscope insertion shaft having the above characteristics and which allows for the flexibility of the shaft to be more easily varied for endoscopes to be used in different applications.

Still yet a further object of the present invention is to provide a flexible endoscope insertion shaft having the above characteristics and which allows for the flexibility of different portions of the same shaft to be varied.

These and other objects of the present invention are achieved by provision of a flexible endoscope insertion shaft having a hollow inner shaft portion formed from a superelastic metal alloy, The hollow inner shaft portion includes a plurality of longitudinally and radially spaced apart holes passing through a wall thereof. Flexibility properties of the insertion shaft are variable without varying the thickness of or the composition of the wall of the hollow inner shaft portion by varying a number of, size of, and/or relative position of the plurality of holes. A polymeric coating coats a surface of the hollow inner shaft portion.

In some embodiments, the hollow inner shaft portion is formed from a nickel-titanium alloy and preferably exhibits both shape memory and superelasticity properties.

In some embodiments the polymeric coating comprises an inner coating layer coating an interior surface of the inner shaft portion, an outer coating layer coating an exterior surface of the inner shaft portion, and portions filling spaces defined by the plurality of holes in the inner shaft portion such that the inner shaft portion is completely embedded within the polymeric coating. In certain of these embodiments, the polymeric coating provides the insertion shaft with inner and outer surfaces which are smooth and devoid of holes in order to facilitate insertion of the insertion shaft into patients and insertion of various components within the insertion shaft. In some embodiments, the polymeric coating is formed from polyurethane. Preferably, flexibility properties of the insertion shaft are variable by varying a thickness of the polymeric coating and/or by varying a material used to form the polymeric coating.

In some embodiments, the plurality of holes in the inner shaft portion are equally spaced along an entire length of the inner shaft portion. In other embodiments, the plurality of holes in the inner shaft portion are spaced more closely in some localized areas along a length of the inner shaft portion than in other localized areas. In some embodiments, the plurality of holes in the inner shaft portion are equally sized. In other embodiments, some of the plurality of holes in the inner shaft portion are larger than others of the plurality of holes. In certain embodiments, the plurality of holes are arranged in four longitudinal lines each line radially spaced apart by about 90°.

The invention and its particular features and advantages will become more apparent from the following detailed description considered with reference to the accompanying drawings.

### Brief Description of the Drawings

**Figure 1** is a side partially cross-sectional side view of a flexible endoscope incorporating a flexible endoscope insertion shaft in accordance with an embodiment of the present invention;

**Figure 2** is an enlarged, partially cross-sectional side view of a portion of the flexible endoscope insertion shaft of Figure 1;

**Figure 3** is an enlarged cross-sectional end view of the flexible endoscope insertion shaft taken along line A-A of Figure 1;

**Figure 4** is an enlarged, partially cross-sectional view of a portion of a flexible endoscope insertion shaft similar to Figure 2, but showing another embodiment of the present invention;

**Figure 5** is an enlarged, partially cross-sectional view of a portion of a flexible endoscope insertion shaft similar to Figure 2, but showing another embodiment of the present invention; and

**Figure 6** is an enlarged, partially cross-sectional view of a portion of a flexible endoscope insertion shaft similar to Figure 2, but showing another embodiment of the present invention.

### Detailed Description of an Embodiment of the Invention

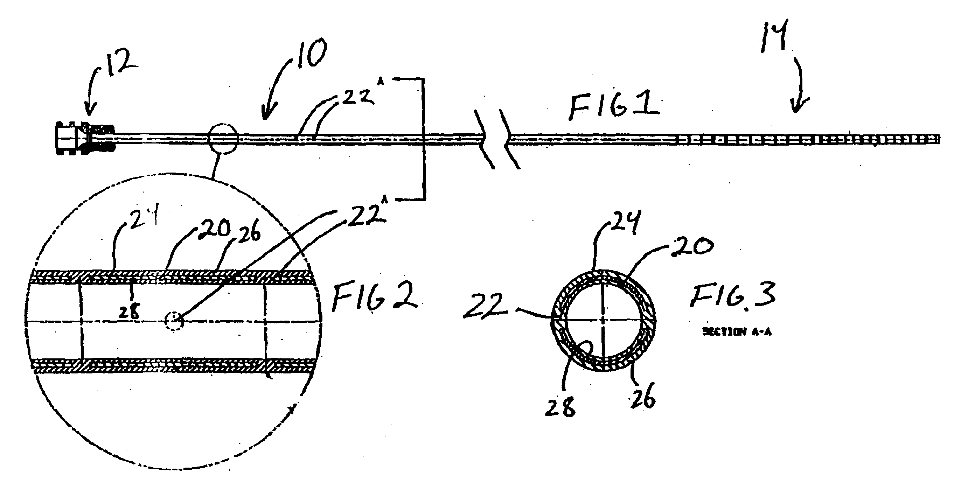

Referring first to Figure 1, a flexible endoscope insertion shaft 10 is shown. Shaft 10 may comprise part of an endoscope which includes various connections, interfaces, end fittings or the like 12 and may or may not include an articulation mechanism 14. In addition, various endoscope components (not shown), such as lumens, lens systems, fiber optic cables, working channels, etc. may be disposed within shaft 10. As such endoscope parts and components are well known in the art, they are not described in detail herein.

As best seen in Figures 2 and 3, shaft 10 includes a hollow inner shaft portion 20. Inner shaft portion 20 is formed from a superelastic metal alloy, such as a nickel-titanium alloy (also known as Nitinol), which exhibits both shape memory and superelasticity properties. Shaft 10 may be configured so as to be substantially straight in a relaxed state (as shown in the Figures), in which case inner shaft portion 20 is substantially cylindrical, or may be configured to be curved or bent in a relaxed state.

Inner shaft portion 20 includes a plurality of longitudinally and radially spaced apart holes 22 passing through the wall thereof. The number of, size of, and relative position of holes 22 may be varied so as to vary the flexibility properties of shaft 10 without varying the thickness of or the composition of the wall of inner shaft portion 20. Holes may be positioned in any of an infinite number of possible configurations. Figures 1-3 show four longitudinal lines of holes 22 each radially spaced apart by about 90° with each hole 22 in each adjacent line of holes 22 being staggered. It should also be noted that Figures 1-3 show a regular pattern of identical holes 22 extending along substantially the entire length of inner shaft portion 20. It should be understood by those skilled in that art that such a configuration would provide a shaft which exhibits substantially similar flexibility properties along its entire length and in all four primary directions (i.e., up, down, left and right).

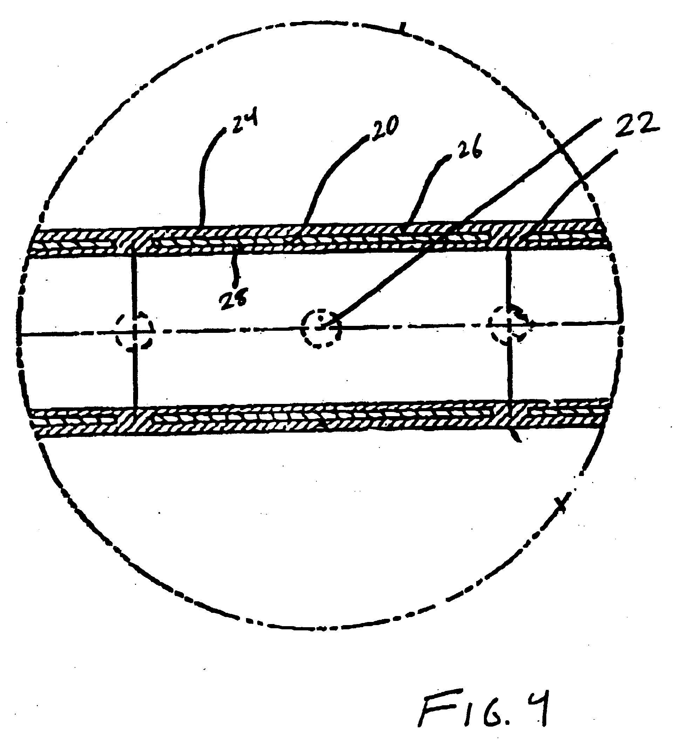

However, it should be understood that this configuration is exemplary, and not intended in any way to be limiting, and that the number of, size of, and relative position of holes 22 can be varied in different localized sections of inner shaft portion 20 in order to provide sections in shaft 10 having varying flexibility properties. For example, Figure 4 shows the case where there are twice as many holes 22 along side surfaces of inner shaft portion 20 as along top and bottom surfaces thereof. It should be understood by those skilled in that art that such a configuration would provide a shaft which exhibits substantially similar flexibility properties along its entire length, but different flexibility properties bending upward or downward as compared to bending leftward or rightward.

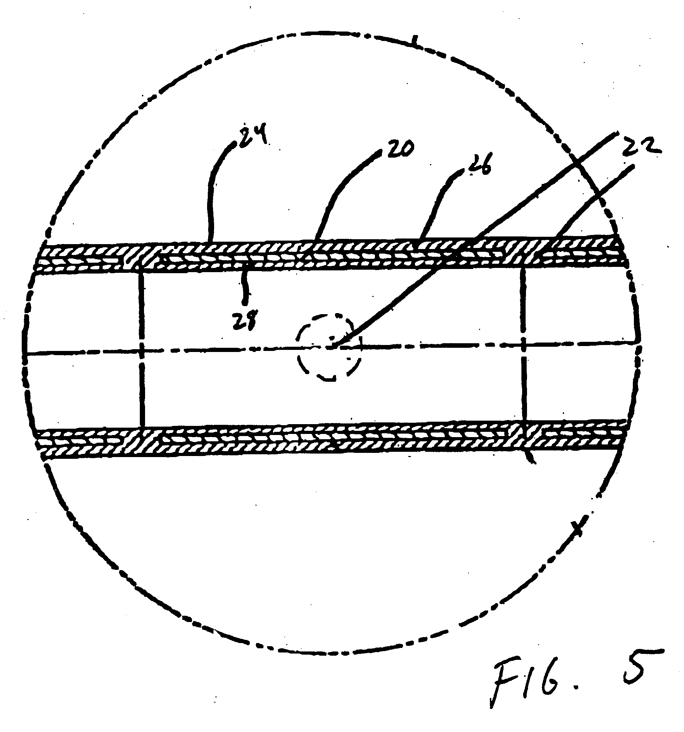

Figure 5 shows another exemplary embodiment similar to Figure 2 with a regularly spaced pattern of staggered holes 22. However, unlike the embodiment shown in Figure 2, in the embodiment shown in Figure 5 the holes 22 along side surfaces of inner shaft portion 20 are significantly larger than the holes 22 along top and bottom surfaces thereof. It should be understood by those skilled in that art that such a configuration would provide a shaft which exhibits substantially similar flexiblllty properties along its entire length, but different flexibility properties bending upward or downward as compared to bending leftward or rightward.

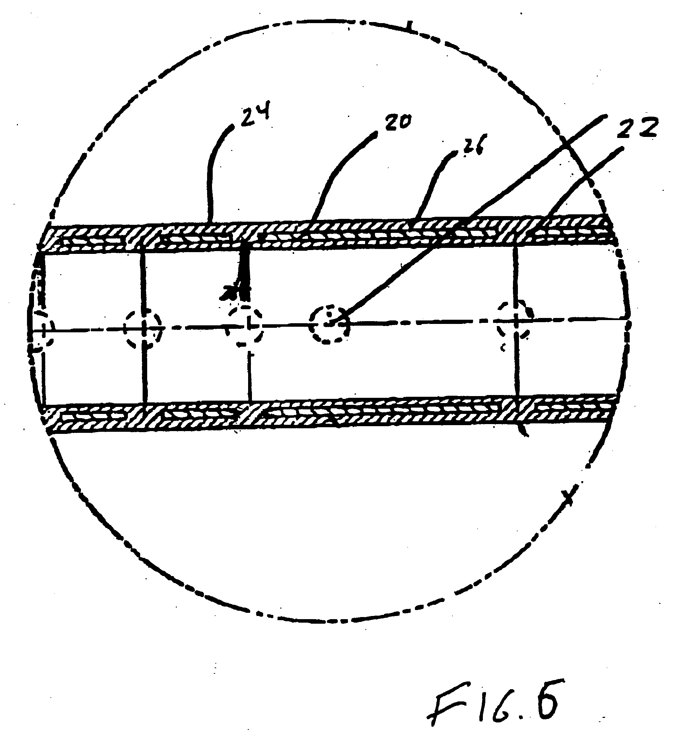

Figure 6 shows another exemplary embodiment of the present invention. In this embodiment, there are a greater number of holes 22 in inner shaft portion to the left of a centerline of the Figure as compared to the right of the centerline. It should be understood by those skilled in that art that such a configuration would provide a shaft which exhibits substantially similar localized flexibility properties bending upward or downward as compared to bending leftward or rightward, but different flexibility properties along the length thereof.

It should be understood than numerous possibilities for creating varying flexibility configurations are possible.

Inner shaft portion 20 is completely encapsulated and embedded within a polymeric coating 24, which includes an inner coating layer 26 on the interior surface of inner shaft portion 20 and an outer coating layer 28 on the exterior surface of inner shaft portion 20. Polymeric coating 24 also fills the spaces defined by holes 22. Polymeric coating 24 provides shaft 10 with inner and outer surfaces which are smooth and devoid of holes, which facilitates insertion of shaft 10 into patients and insertion of various components within shaft 10. Any of numerous materials may be used to form polymeric coating 24, although it has been found that polyurethane provides acceptable results. It should be understood that the flexibility properties of shaft 10 can be varied by varying the thickness of inner coating layer 26 and/or outer coating layer 28, and/or by varying the material used to form polymeric coating 24. Although the Figures show outer coating layer 28 having a thickness greater than inner coating layer 26, it should be understood that the reverse is also possible, or that both may have the same thickness.

The present invention, therefore, provides a flexible endoscope insertion shaft which is flexible, torsionally stable and crush resistant, which may be guided around relatively small radii of curvature, which is capable of elastically returning to its original position when removed from a patient's body, which is not prone to being crushed, kinked or the like, which does not require that the dimensions and/or composition of the shaft to be carefully controlled in order to achieve the desired flexibility and kink resistance characteristics, which allows for the flexibility of the shaft to be more easily varied for endoscopes to be used in different applications, and which allows for the flexibility of different portions of the same shaft to be varied.

Although the invention has been described with reference to a particular arrangement of parts, features and the like, these are not intended to exhaust all possible arrangements or features, and indeed many other modifications and variations will be ascertainable to those of skill in the art.

## Claims

1. A flexible endoscope insertion shaft comprising:
a hollow inner shaft portion (20) formed from a superelastic metal alloy, said hollow inner shaft portion (20) including a plurality of longitudinally and radially spaced apart holes (22) passing through a wall thereof, wherein flexibility properties of said insertion shaft (10) are variable without varying the thickness of or the composition of the wall of said hollow inner shaft portion (20) by varying a number of, size of, and/or relative position of the plurality of holes (22); and
a polymeric coating (24) which coats a surface of said hollow inner shaft portion (20).

2. The flexible endoscope insertion shaft of claim 1, wherein said hollow inner shaft portion (20) is formed from a nickel-titanium alloy.

3. The flexible endoscope insertion shaft of claim 1 or 2, wherein said hollow inner shaft portion (20) exhibits both shape memory and superelasticity properties.

4. The flexible endoscope insertion shaft of anyone of claims 1 through 3, wherein said polymeric coating (24) comprises an inner coating layer (26) coating an interior surface of said inner shaft portion (20), an outer coating layer (28) coating an exterior surface of said inner shaft portion (20), and portions filling spaces defined by the plurality of holes (22) in said inner shaft portion (20) such that said inner shaft portion (20) is completely embedded within said polymeric coating.

5. The flexible endoscope insertion shaft of claim 4, wherein said polymeric coating (24) provides said insertion shaft (10) with inner and outer surfaces which are smooth and devoid of holes in order to facilitate insertion of said insertion shaft (10) into patients and insertion of various components within said insertion shaft (10).

6. The flexible endoscope insertion shaft of anyone of claims 1 through 5, wherein said polymeric coating (24) is formed from polyurethane.

7. The flexible endoscope insertion shaft of anyone of claims 1 through 6, wherein flexibility properties of said insertion shaft (10) are variable by varying a thickness of said polymeric coating (24) and/or by varying a material used to form said polymeric coating (24).

8. The flexible endoscope insertion shaft of anyone of claims 1 through 7, wherein the plurality of holes (22) in said inner shaft portion (20) are equally spaced along an entire length of said inner shaft portion (20).

9. The flexible endoscope insertion shaft of anyone of claims 1 through 8, wherein the plurality of holes (22) in said inner shaft portion (20) are spaced more closely in some localized areas along a length of said inner shaft portion (20) than in other localized areas.

10. The flexible endoscope insertion shaft of anyone of claims 1 through 9, wherein the plurality of holes (22) in said inner shaft portion (20) are equally sized.

11. The flexible endoscope insertion shaft of anyone of claims 1 through 10, wherein some of the plurality of holes (22) in said inner shaft portion (20) are larger than others of the plurality of holes (22).

12. The flexible endoscope insertion shaft of anyone of claims 1 through 11, wherein the plurality of holes (22) are arranged in four longitudinal lines each line radially spaced apart by about 90°.

13. An endoscope, comprising a flexible insertion shaft according to anyone of claims 1 through 12.
